# EUROPÄISCHE PATENTSCHRIFT

(11) **EP 0 586 998 B1**
(45) Veröffentlichungstag und Bekanntmachung des Hinweises auf die Patenterteilung: **07.01.1998**
(21) Anmeldenummer: 93113802.8
(22) Anmeldetag: 30.08.1993
(51) Int. Cl.: C02F 1/02, C02F 1/72, C02F 11/08

(54) **Verfahren zur Behandlung von organischen Stoffen, insbesondere chlororganische Verbindungen enthaltenden Abwässern aus der Epichlorhydrinherstellung**
Process for the treatment of waste water containing organic matter, especially chlorinated organic compounds from the production of epichlorohydrine
Procédé pour le traitement des eaux usées contenant des composés organiques, en particulier des composés chloroorganiques lors de la fabrication de l'épichlorhydrine

(30) Priorität: 06.09.1992 DE 4229355; 06.09.1992 DE 4229356
(43) Veröffentlichungstag der Anmeldung: 16.03.1994
(73) Patentinhaber: Solvay Deutschland GmbH, D-30173 Hannover (DE)
(72) Erfinder: Dilla, Wolfgang, D-4134 Rheinberg 1 (DE); Dillenburg, Helmut, D-4134 Rheinberg 1 (DE); Plönissen, Erich, D-4134 Rheinberg 1 (DE); Sell, Michael, D-3150 Peine (DE)
(74) Vertreter: Lauer, Dieter, Dr.

(56) Entgegenhaltungen:
- EP-A- 0 454 642
- WO-A-92/05118
- FR-A- 2 332 959
- DATABASE WPI Section Ch, Week 7609, Derwent Publications Ltd., London, GB; Class D15, AN 76-15946X & JP-A-51 005 864 (CHISSO CORP) 19. Januar 1976
- DATABASE WPI Section Ch, Week 8832, Derwent Publications Ltd., London, GB; Class D15, AN 88-223735 & JP-A-63 158 188 (SUMITOMO METAL & TOA GOSEI CHEM) 1. Juli 1988

## Beschreibung

Die vorliegende Erfindung betrifft ein Verfahren zur Behandlung von organische Stoffe, insbesondere chlororganische Verbindungen enthaltenden Abwässern aus der Epichlorhydrinherstellung, wobei das Epichlorhydrin durch Umsetzung von Dichlorpropanol mit mindestens einer alkalisch wirkenden Verbindung, vorzugsweise einer calciumhydroxidhaltigen wäßrigen Lösung oder Suspension, erhalten und durch Destillation aus dem Reaktionsgemisch abgetrennt wird und als Sumpfprodukt ein Abwasser zurückbleibt, das gesättigte und/oder ungesättigte aliphatische und/oder alicyclische Chlor-Kohlenwasserstoffe, -Ether, -Alkohole, -Ketone, -Aldehyde und/oder -Carbonsäuren enthält.

Die technische Herstellung von Epichlorhydrin (1-Chlor-2,3-epoxy-propan) basiert nach bekannten einschlägigen Verfahren auf der Dehydrochlorierung von Dichlorpropanol mit alkalisch wirkenden Mitteln, vorzugsweise wäßrigen Lösungen oder Suspensionen von Calciumhydroxid oder Natriumhydroxid, bei erhöhten Temperaturen. Der Ausgangsstoff Dichlorpropanol wird vorzugsweise als wäßrige Lösung des Isomerengemisches 1,3- und 2,3-Dichlorpropanol durch Umsetzung von Allylchlorid, Chlor und Wasser erhalten.

Das gemäß dem vorgenannten Verfahren gebildete Epichlorhydrin wird aus dem Reaktionsgemisch durch Destillation, vorzugsweise Dampfdestillation abgetrennt. Dabei tritt als Sumpfprodukt aus dem Synthesereaktor eine wäßrige Lösung oder Suspension aus, die neben geringen Mengen des Reaktionsproduktes weitere organische, insbesondere chlororganische, und anorganische Verbindungen als Nebenprodukte der Synthese sowie nicht umgesetzte Ausgangsstoffe enthält. Dieses als Abwasser anfallende Sumpfprodukt weist (bei Einsatz von Calciumhydroxid als alkalisch wirkendes Mittel in der Epichlorhydrinsynthese) typischerweise folgende Verbindungen auf: chlorierte cyclische oder acyclische Alkane und Alkene, gesättigte und/oder ungesättigte aliphatische und/oder alicyclische Chlor-Ether, -Alkohole, -Ketone, -Aldehyde und/oder -Carbonsäuren sowie, neben weiteren zum CSB (chemischer Sauerstoffbedarf) des Abwassers beitragenden Verbindungen, insbesondere Glycerin und Glycerinderivate, des weiteren Calciumchlorid, Calciumcarbonat und gegebenenfalls im Überschuß eingesetztes Calciumhydroxid.

Die in dem Sumpfprodukt enthaltenen chlororganischen Verbindungen tragen zum Summenparameter AOX (Adsorbierbare Organische Halogen-Verbindungen) des Abwassers bei. Der AOX wird als der Teil organischer Halogenverbindungen (X = F, Cl, Br, J) bestimmt, die sich an Aktivkohle adsorbieren lassen, wobei die gesamte adsorbierte Menge auf X = Cl umgerechnet wird.

Derartige, halogenierte organische Verbindungen enthaltende Abwässer stellen ein besonderes Problem in der Abwasserreinigung dar, da die Entfernung dieser Stoffe wegen der hohen Stabilität der kovalenten Kohlenstoff-Halogen-Bindungen, insbesondere bei sp²-gebundenen Halogenen, technisch sehr aufwendig und damit häufig unwirtschaftlich ist.

Bekannte Maßnahmen zur Reduzierung chlorierter und anderer halogenierter organischer Stoffe in Abwässern sind chemisch-physikalische sowie biotechnologische Verfahren.

Der Abbau von halogenierten organischen Verbindungen in der biochemischen Reinigungsstufe einer Kläranlage wirft verschiedene Probleme auf: Einerseits sind viele dieser Verbindungen nur schwer oder gar nicht einer biologischen Zersetzung durch Mikroorganismen zugänglich. Andererseits dürfen die Einsatzkonzentrationen an AOX-erzeugenden Stoffen im Abwasser nicht hoch sein und sollten außerdem weitgehend konstante Werte aufweisen. Zudem ist das Volumen des Belebtschlamms in solchen Anlagen groß und die Anreicherung der organischen Halogenverbindungen im Schlamm stellt ein weiteres Problem dar.

Im Stand der Technik werden daher bevorzugt Verfahren zur chemisch-physikalichen Entfernung von halogenorganischen Verbindungen aus Abwässern vorgeschlagen, wobei diese Verfahren zur Haupt- oder Vorreinigung (mit nachfolgender biochemischer Behandlung) des Abwassers eingesetzt werden.

Methoden, die hier zur Verfügung stehen, sind beispielsweise die Aktivkohlereinigung sowie spezielle Extraktionsverfahren. Nachteil dieser Verfahren ist, daß sie ein mit halogenierten organischen Verbindungen belastetes Sekundärprodukt (beladene Aktivkohle bzw. Extraktionsmittel) erzeugen.

Vielfach angewandte Maßnahmen zur Vernichtung halogenorganischer Verbindungen in Abwässern stellen die chemisch-thermischen Verfahren dar. Hierzu gehören die sogenannten naßoxidativen Verfahren, bei denen in einer oxidierenden Atmosphäre bei hohen Temperaturen und erheblichen Drücken eine Zersetzung halogenierter organischer Verbindungen durchgeführt wird. Diese Methode ist zwar sehr effektiv, jedoch auch kostenintensiv wegen ihres hohen Energieverbrauchs und der aufwendigen Apparaturen.

Um die extremen physikalischen Bedingungen chemisch-thermischer Verfahren zu mildern, wird im Stand der Technik der Einsatz katalytisch wirkender Verbindungen vorgeschlagen, wobei derartige Stoffe entweder durch Zugabe entsprechender Reagenzien in das zu dehalogenierende System gelangen können oder sich während der Zersetzungsreaktion als Zwischenprodukte bilden.

Als Stoffe, die gegenüber organisch gebundenen Halogenen eine hohe Reaktivität aufweisen, werden beispielsweise bestimmte Metalle, Metallhydride oder Metallalkoholate, allein oder in Verbindung mit einer starken anorganischen Base, eingesetzt.

Nachteil der bekannten chemisch-physikalischen Verfahren zur Zersetzung bzw. Vernichtung halogenierter organischer Verbindungen sind ihre verhältnismäßig hohen Kosten, die insbesondere durch den Verbrauch an teuren Reagenzien und durch die Einstellung einer oxidierenden oder inerten Atmosphäre sowie relativ hoher Temperaturen und Drücke und die damit verbundene Notwendigkeit aufwendiger Apparaturen entstehen. Zudem wirken sich in Wirtschaftlichkeitsrechnungen der bekannten Methoden zur Dehalogenierung und/oder Dehydrohalogenierung halogenorganischer Verbindungen die vielfach langen Reaktionszeiten (oft mehr als zehn Stunden) und die häufig nur mäßigen Abbauraten ungünstig aus.

Weiterhin sind Verfahren zur Behandlung von Abwässern aus der Zellstoffbleiche bekannt, bei denen die im Abwasser enthaltenen Chlorligninverbindungen unter Einhaltung bestimmter Temperaturen, pH-Werte und Verweilzeiten partiell dehalogeniert und/oder dehydrohalogeniert werden. Entsprechend ist aus der DE-OS 36 20 980 ein aufwendiges dreistufiges Verfahren bekannt, das eine Vorbehandlung des Abwassers mit Fällungsmethoden vorsieht und dessen thermische Hydrolysestufe durch eine pH-Einstellung auf 11,5 mit Kalkmilch und/oder NaOH, eine Temperatureinstellung auf 40 bis 70°C und eine Verweilzeit von 1 bis 3 Stunden gekennzeichnet ist. Gemaß einer weiteren Patentanmeldung, WO 92/05118, wird der Abbau von Chlorligninverbindungen bei pH-Werten von 6 bis 11, Temperaturen von 90 bis 150°C, einem Überdruck von 70 bis 475 kPa in einer Verweilzeit 2 bis 5 Minuten durchgeführt, wobei Jedoch AOX-Abbauraten von mehr als 60 % vorzugsweise unter Einsatz weiterer chemischer Reagentien oder durch gezieltes Vermischen verschiedener Abwässer aus der Zellstoffbleiche (wobei vermutlich katalytische Prozesse eine Rolle spielen) erreicht werden.

Aus der FR-A-2332959 ist bekannt, daß thermisch-alkalisch vorbehandeltes Abwasser mit Natriumhypochlorit oder H₂O₂ nachbehandelt werden kann. Wenn halogenorganische Verbindungen im Abwasser vorhanden sind, findet die hydrolytische Behandlung in Gegenwart von Stickstoffverbindungen statt.

Die JP-A-51005864 offenbart ein katalytisches Verfahren zur Behandlung von Abwässern in Gegenwart von Wasserstoff, ohne daß eine thermisch-alkalische Vorbehandlung vorgeschrieben ist.

Die hier vorgeschlagenen Verfahren enthalten keine Hinweise auf ihren möglichen Einsatz in der Behandlung von Abwässern aus der Epichlorhydrinsynthese. Zudem ist eine Anwendung der vorgenannten Methoden zum Abbau von Chlorligninverbindungen in Abwässern aus der Zellstoffbleiche wegen der völlig anderen Abwasserzusammensetzung und den damit nicht übertragbaren Parametern hinsichtlich pH-Wert, Temperatur, Druck und Verweilzeit in einem Behandlungsverfahren für Abwässer aus der Epichlorhydrinsynthese unmöglich, wobei insbesondere AOX-Abbauraten von über 50 % auf diese Weise nicht erzielt werden können.

Nach Bewertung des vorgenannten Standes der Technik sollte ein wirtschaftliches Verfahren zur Entfernung von halogenierten organischen Verbindungen aus industriellen Abwässern sich durch folgende Merkmale auszeichnen: geringen technischen Aufwand in der Durchführung des Verfahrens, möglichst einfache Reaktionsführung, geringer Verbrauch an Chemikalien sowie hohe AOX-Abbauraten bei niedrigen Temperaturen und Drücken sowie geringen Verweilzeiten.

Insbesondere für die Behandlung von Abwässern aus der Epichlorhydrinsynthese konnte ein solches Verfahren bisher nicht bereitgestellt werden.

Aufgabe der vorliegenden Erfindung war es daher, ein Verfahren zur Dechlorierungs- und/oder Dehydrochlorierungsbehandlung von mit chlororganischen Stoffen belasteten Abwässern aus der Epichlorhydrinsynthese bereitzustellen, das sich durch eine einfache Verfahrensführung, geringen energetischen und apparativen Aufwand sowie einen geringen Einsatz an zusätzlichen Reagentien auszeichnet, so daß die Abwasserprobleme des bekannten Verfahrens der Epichlorhydrinsynthese gelöst werden können und somit insgesamt ein wirtschaftliches, großtechnisch durchführbares und ökologisch vertretbares Herstellungsverfahren, das einen Reinigungsprozeß für die Aufarbeitung von prozeßbedingten Rückständen einschließt, zur Verfügung gestellt wird.

Es besteht bereits ein dringendes Bedürfnis, die chlororganischen Verbindungen, die im Abwasser einer Epichlorhydrinsyntheseanlage anfallen, zumindest teilweise zu entfernen, um damit ökologischen Zielsetzungen gerecht zu werden. Dieses Bedürfnis ist in verschiedenen Patentanmeldungen auf diesem Fachgebiet dokumentiert, wie beispielsweise in der EP-A 0 247 670, der DE-OS 30 16 667 und der DE-OS 35 20 019. Die hier vorgeschlagenen Verfahren umfassen entweder aufwendige Reinigungsstufen zur Abtrennung der unerwünschten chlororganischen Verbindungen, oder es wird das Verfahren der Epichlorhydrinherstellung so weit modifiziert, daß der Anfall an chlororganischen Nebenprodukten minimiert werden kann. In jedem Fall handelt es sich aber um Maßnahmen, die kostenintensiv sind und vielfach nur zu einer unbefriedigenden Verminderung der organischen Chlorverbindungen im Abwasser führen.

Gegenstand der vorliegenden Erfindung ist daher ein Verfahren, wobei das aus dem Reaktionsbehälter austretende oder ausgetragene Abwasser, das adsorbierbare organische Halogenverbindungen (AOX) in einer Menge von
mehr als 10 mg/l, vorzugsweise
mehr als 20 mg/l,
enthält, einer mindestens zweistufigen Dechlorierungs- und/oder Dehydrochlorierungsbehandlung unterworfen wird, indem zunächst in einem ersten Schritt das Abwasser, das einen pH-Wert von
10 bis 14, vorzugsweise
11 bis 13,
(gemessen bei Raumtemperatur) aufweist oder auf einen solchen pH-Wert eingestellt wird, in mindestens einen Reaktor eingebracht wird und/oder diesen durchläuft, wobei eine Temperatur von
mehr als 75°C, vorzugsweise
mehr als 85°C,
ein Druck von
mindestens 1 bar (absolut), vorzugsweise
mindestens 2 bar (absolut),
und eine Verweilzeit von
mindestens 0,5 Stunden, vorzugsweise
mindestens 1 Stunde,
in dem Reaktor eingestellt oder eingehalten werden und die in dem Abwasser enthaltenen chlororganischen Verbindungen teilweise dechloriert und/oder dehydrochloriert werden und anschließend in einem zweiten Schritt mindestens eine weitere Dechlorierungs- und/oder Dehydrochlorierungsbehandlung durchgeführt wird, vorzugsweise in Gegenwart eines wasserstoffhaltigen Gases oder einer Wasserstoff freisetzenden Verbindung und/oder in Gegenwart einer katalytisch wirkenden Substanz, und nachfolgend das behandelte Abwasser aus dem Reaktor oder den Reaktoren ausgetragen und/oder einer biologischen Behandlung unter Verwendung von Mikroorganismen, unterworfen wird.

Das erfindungsgemäße Verfahren ermöglicht es, auf wirtschaftliche Weise chlororganische Verbindungen im Abwasserstrom einer Epichlorhydrinsyntheseanlage mit einer hohen Abbaurate großtechnisch zu dechlorieren und/oder zu dehydrochlorieren, so daß die ökologischen und technischen Probleme, die bisher bei der Entsorgung dieses Abwassers aufgetreten sind, weitgehend gelöst werden können.

Gegenüber bekannten Verfahren zur Entfernung von halogenorganischen Verbindungen aus Abwässern ist das erfindungsgemäße Verfahren apparativ einfacher gestaltet, verbraucht weniger Energie und vermeidet gegebenenfalls in dem ersten Verfahrensschritt den Einsatz zusätzlicher Reagenzien. Außerdem fallen keine sicherheitstechnisch und/oder ökologisch bedenklichen Sekundärprodukte an, die anschließend wiederum aufgearbeitet beziehungsweise entsorgt werden müßten.

Insbesondere wurde gefunden, daß durch die Auswahl der erfindungsgemäßen Parameter Temperatur, Druck und Verweilzeit des alkalischen Abwassers aus der Epichlorhydrinsynthese, das üblicherweise pH-Werte von 11,5 bis 12,5 aufweist, gegebenenfalls ohne weitere pH-Anhebung (durch entsprechende Zugabe basisch wirkender Verbindungen) dem erfindungsgemäßen thermisch-alkalischen Dechlorierungs- bzw. Dehydrochlorierungsverfahren gemäß dem ersten Verfahrensschritt unterworfen werden kann, wobei trotz dieser relativ niedrigen pH-Werte überraschenderweise hohe AOX-Abbauraten erreicht werden.

Durch die erfindungsgemäße, mindestens zweistufige Dechlorierungs- und/oder Dehydrochlorierungsbehandlung des Abwassers läßt sich auf kostengünstige Weise gegebenenfalls ein nahezu vollständiger AOX-Abbau durchführen, indem bereits im ersten Schritt mit begrenztem energetischen und technischen Aufwand eine Teildechlorierung und/oder -dehydrochlorierung der in dem Abwasser enthaltenen chlororganischen Verbindungen vorgenommen wird und anschließend unter Einsatz relativ geringer Mengen an zusätzlichen Reagentien eine weitergehende Dechlorierung und/oder Dehydrochlorierung durchgeführt wird, wobei der AOX-Abbau vorzugsweise in dem Maß erfolgt, daß schließlich ein Abwasser erhalten wird, das entweder nur noch sehr geringe Mengen an AOX-erzeugenden Komponenten (vorzugsweise unter 10 mg/l) aufweist oder in dem der Gehalt an AOX-erzeugenden Stoffen so weit reduziert ist, daß es problemlos einer weiteren, biologischen Behandlung unter Verwendung von Mikroorganismen unterzogen werden kann, um letztendlich auch die verbleibenden organischen Verbindungen im Abwasser abzubauen.

Ein weiterer Vorteil des erfindungsgemäßen Verfahrens ist, daß zu seiner Durchführung prinzipiell alle im Stand der Technik gebräuchlichen Reaktoren eingesetzt werden können, wobei keine besonderen Maßnahmen hinsichtlich der Ausführung der Reaktorwände und der gegebenenfalls vorhandenen Einbauten vorgenommen werden müssen, da bei dem erfindungsgemäßen Verfahren weder Verkrustungs- noch Korrosionsprobleme auftreten.

Es war weiterhin überraschend, daß trotz der im Vergleich mit bekannten Verfahren "milden" Bedingungen des erfindungsgemäßen Verfahrens, wie den relativ niedrigen Temperaturen und Drücken sowie kurzen Verweilzeiten, die chlororganischen Rückstände bzw. Nebenprodukte aus der Epichlorhydrinsynthese weitgehend chemisch-thermisch abgebaut werden, so daß der AOX eines Abwassers aus der Epichlorhydrinsynthese (gemäß dem bekannten Verfahren), der typischerweise Werte zwischen 25 und 45 mg/l aufweist, auf unter 10 mg/l, vorzugsweise unter 5 mg/l, gesenkt werden kann und die nach der Dechlorierungs- und/oder Dehydrochlorierungsbehandlung zurückbleibende, gegebenenfalls neutralisierte, calciumchloridhaltige wäßrige Lösung somit künftigen gesetzlichen Bestimmungen hinsichtlich des AOX-Gehaltes insustrieller Abwässer genügt.

Die Eliminierung AOX-erzeugender Komponenten zu mehr als 75 %, vorzugsweise mehr als 97 %, im Abwasser aus der Epichlorhydrinsynthese wird insbesondere durch die vorteilhaften Ausführungsformen des erfindungsgemäßen Verfahrens hinsichtlich der Einstellung von pH-Wert, Temperatur, Druck und Verweilzeit erreicht, wodurch eine gezielte Steuerung der AOX-Abbauraten möglich ist.

Danach weist in dem ersten Verfahrensschritt das zu behandelnde Abwasser gemaß einer ersten vorzugsweisen Ausführungsform des erfindungsgemäßen Verfahrens einen pH-Wert von 11,5 bis 12,5 (gemessen bei Raumtemperatur) auf oder wird darauf eingestellt, und die Dechlorierungs- und/oder Dehydrochlorierungsbehandlung der in dem Abwasser enthaltenen chlororganischen Verbindungen wird in dem Reaktor bei einer Temperatur von 125 bis 135 °C, einem Druck von 2,5 bis 4,0 bar (abs.) und einer Verweilzeit des Abwassers von 1 bis 7 Stunden durchgeführt.

Nach einer weiteren vorteilhaften Ausführungsform des erfindungsgemäßen Verfahrens weist in dem ersten Verfahrensschritt das zu behandelnde Abwasser einen pH-Wert von 13 bis 14 (gemessen bei Raumtemperatur) auf oder wird darauf eingestellt, und die Dechlorierungs- und/oder Dehydrochlorierungsbehandlung der in dem Abwasser enthaltenen chlororganischen Verbindungen wird in dem Reaktor bei einer Temperatur von 125 bis 135°C, einem Druck von 2,5 bis 4,0 bar (absolut) und einer Verweilzeit des Abwassers von 1 bis 4 Stunden durchgeführt.

Nach einer weiteren vorteilhaften Ausführungsform des erfindungsgemäßen Verfahrens weist in dem ersten Verfahrensschritt das zu behandelnde Abwasser einen pH-Wert von 11,5 bis 12,5 (gemessen bei Raumtemperatur) auf oder wird darauf eingestellt, und die Dechlorierungs- und/oder Dehydrochlorierungsbehandlung der in dem Abwasser enthaltenen chlororganischen Verbindungen wird in dem Reaktor bei einer Temperatur von 175 bis 185°C, einem Druck von 9,0 bis 10,5 bar (absolut) und einer Verweilzeit des Abwassers von 1 bis 8 Stunden durchgeführt.

Nach einer weiteren vorteilhaften Ausführungsform des erfindungsgemäßen Verfahrens weist in dem ersten Verfahrensschritt das zu behandelnde Abwasser einen pH-Wert von 11,5 bis 12,5 (gemessen bei Raumtemperatur) auf oder wird darauf eingestellt, und die Dechlorierungs- und/oder Dehydrochlorierungsbehandlung der in dem Abwasser enthaltenen chlororganischen Verbindungen wird in dem Reaktor bei einer Temperatur von 85 bis 90°C, einem Druck von 1,0 bis 1,5 bar (absolut) und einer Verweilzeit des Abwassers von 1 bis 7 Stunden durchgeführt.

Nach einer weiteren vorteilhaften Ausführungsform des erfindungsgemäßen Verfahrens weist in dem ersten Verfahrensschritt das zu behandelnde Abwasser einen pH-Wert von 11,5 bis 12,5 (gemessen bei Raumtemperatur) auf oder wird darauf eingestellt, und die Dechlorierungs- und/oder Dehydrochlorierungsbehandlung der in dem Abwasser enthaltenen chlororganischen Verbindungen wird in dem Reaktor bei einer Temperatur von 155 bis 165 °C, einem Druck von 5,0 bis 7,4 bar (abs.) und einer Verweilzeit des Abwassers von 4 bis 8 Stunden durchgeführt.

Erfindungsgemäß wurde festgestellt, daß auch bei relativ niedrigen Temperaturen und Drücken sowie einer pH-Einstellung vorzugsweise im Bereich von 11,5 bis 12,5 hohe Abbauraten mit Verweilzeiten unter 10 Stunden möglich sind, was einen einen weiteren Vorteil des erfindungsgemäßen Verfahrens darstellt.

Es hat sich weiterhin als vorteilhaft herausgestellt, bei der Epichlorhydrinsynthese als calciumhydroxidhaltige wäßrige Lösung oder Suspension Kalkmilch mit einem Überschuß an Calciumhydroxid (bezogen auf die zur vollständigen Umsetzung theoretisch berechnete stöchiometrische Menge an Dichlorpropanol) einzusetzen, wobei die überschüssige Menge so groß gewählt wird, daß das zu behandelnde Abwasser bereits durch den das Abwasser erzeugenden Prozeß auf pH-Werte von 11 bis 12,5 (gemessen bei Raumtemperatur) eingestellt wird und somit schon bei Austritt aus dem Synthesereaktor den zur thermisch-alkalischen Behandlung notwendigen pH-Wert aufweist.

Um das Abwasser gegebenenfalls auf die erfindungsgemäßen pH-Werte (gemessen bei Raumtemperatur) einzustellen, kann auch eine entsprechende Menge Alkali- und/oder Erdalkalihydroxid, vorzugsweise eine wäßrige Natriumhydroxid- und/oder Calciumhydroxidlösung, zugesetzt werden.

Die pH-Wert-Einstellung kann nach einer weiteren vorteilhaften Ausführungsform des erfindungsgemäßen Verfahrens auch mit einer entsprechenden Menge Alkalicarbonat und/oder Alkalihydrogencarbonat, vorzugsweise einer wäßrigen Natriumcarbonat- und/oder Natriumhydrogencarbonatlösung, vorgenommen werden.

Vorteilhafterweise wird das thermisch-alkalisch behandelte Abwasser zur Weiterbehandlung in einen weiteren Reaktor eingeleitet und dort in Gegenwart eines wasserstoffhaltigen Gases oder einer Wasserstoff freisetzenden Verbindung und/oder in Gegenwart einer katalytisch wirkenden Substanz einer weiteren Dechlorierungs- und/oder Dehydrochlorierungsbehandlung unterworfen. Die vorgenannte, weitere Dechlorierungs- und/oder Dehydrochlorierungsbehandlung kann aber auch in dem Reaktor für die thermischalkalische Abwasserbehandlung durchgeführt werden.

Vorzugsweise wird in dem erfindungsgemäßen Verfahren hochreiner Wasserstoff (insbesondere mindestens 99,9 %iger Wasserstoff) als wasserstoffhaltiges Gas eingesetzt. Es kann aber gegebenenfalls auch ein Gemisch von Wasserstoff mit einem Inertgas verwendet werden.

Als katalytisch wirkende Substanz wird vorzugsweise ein Metall, eine Metallegierung und/oder eine anorganische und/oder organische Metallverbindung oder ein Stoffgemisch, das eine oder mehrere dieser Verbindungen enthält, eingesetzt, wobei die katalytisch wirkende Substanz unter Zusatz eines Trägerstoffes, vorzugsweise eines aluminiumoxidhaltigen Trägers, indem sie beispielsweise auf diesen Träger aufgebracht wird, in dem erfindungsgemäßen Vefahren verwendet werden.

Nach einer weiteren vorzugsweisen Ausführungsform des erfindungsgemäßen Verfahrens wird als katalytisch wirkende Substanz eine palladiumhaltige Verbindung eingesetzt.

Da das aus dem Synthesereaktor austretende Abwasser, insbesondere bei überschüssig eingesetzter Kalkmilch als alkalisch wirkendes Mittel in der Epichlorhydrinherstellung, suspendierte Feststoffe, vorzugsweise nichtgelöstes Calciumhydroxid, enthält, was zu Störungen im Behandlungsverfahren beispielsweise durch Bildung von Verstopfungen führen kann, ist es vorteilhaft, das Abwasser gegebenenfalls vor, während und/oder nach der Dechlorierungs- und/oder Dehydrochlorierungsbehandlung von den suspendierten Feststoffen zumindest teilweise zu befreien, indem durch entsprechende, übliche Maßnahmen diese Feststoffe abgetrennt bzw. abgeschieden werden. Dies erfolgt vorzugsweise durch chemische Reaktion, indem beispielsweise suspendiertes Calciumhydroxid durch Zugabe von Salzsäure gelöst wird, und/oder durch mechanische Trennverfahren, wie beispielsweise Filtration oder Sedimentation.

Die Durchführung des erfindungsgemäßen Verfahrens kann im kontinuierlichen oder diskontinuierlichen Betrieb erfolgen, wobei vorzugsweise eine Reaktoreinheit aus mindestens drei, insbesondere vier (Einzel-) Reaktoren, die entweder hintereinander- oder parallelgeschaltet sind, eingesetzt wird.

Wegen der vorgenannten, gegebenenfalls im Abwasser vorhandenen, suspendierten Feststoffe, erfolgt die Einspeisung des zu behandelnden Abwasserstroms vorteilhafterweise am Kopf des Reaktors bzw. der Reaktoren in einer abwärtsgerichteten Strömung und das behandelte Abwasser wird am Boden des Reaktors aus diesem ausgetragen. Eine Einspeisung des Abwassers von unten in das Reaktionsgefäß mit aufwärtsgerichteter Strömung würde zu Verstopfungsproblemen durch die suspendierten Feststoffe führen.

Zur kontinuierlichen Durchführung der Dechlorierungs- und/oder Dehydrochlorierungsbehandlung des Abwassers kann aber auch ein Strömungsrohr oder Rohrreaktor eingesetzt werden, wobei nach einer weiteren vorteilhaften Ausführungsform des erfindungsgemäßen Verfahrens in dem Strömungsrohr oder Rohreaktor eine Strömungsgeschwindigkeit von mehr als 4 m/sec (Meter/Sekunde) eingestellt wird. Vorzugsweise beträgt die Strömungsgeschwindigkeit 8,5 m/sec.

Der zweite Verfahrensschritt des erfindungsgemäßen Verfahrens kann in dem Reaktor oder den Reaktoren, die für den ersten Behandlungsschritt bereits eingesetzt wurden, durchgeführt werden, indem nach Beendigung des beispielsweise in einem diskontinuierlichen Verfahren erfolgten ersten Behandlungsschrittes das vorbehandelte Abwasser in dem Reaktor oder den Reaktoren verbleibt und dort unter Einleitung des Reagens bzw. der Reagentien für die Weiterbehandlung (über entsprechende Zuführeinrichtungen) die weitere Dechlorierungs- und/oder Dehydrochlorierungsbehandlung vorgenommen wird. Ist die thermisch-alkalische Behandlung im kontinuierlichen Betrieb durchgeführt worden, so kann der vorbehandelte Abwasserstrom nach Austritt aus dem Reaktor oder den Reaktoren in diesen bzw. diese zurückgeführt werden, um dort, in einem kontinuierlichen oder diskontinuierlichen Verfahren, der weiteren Dechlorierungs- und/oder Dehydrochlorierungsbehandlung unterworfen zu werden.

Nach einer weiteren Ausführungsform des erfindungsgemäßen Verfahrens kann das in dem ersten Verfahrensschritt behandelte Abwasser aber auch aus dem Reaktor oder den Reaktoren ausgetragen und nachfolgend in mindestens einen weiteren Reaktor zur Behandlung gemäß dem zweiten Verfahrensschritt eingetragen werden.

Für eine katalytische Behandlung des Abwassers in dem zweiten Verfahrensschritt kann hier beispielsweise ein Festbett- oder ein Fließbettreaktor, vorzugsweise ein Wirbelschichtreaktor, eingesetzt werden.

Da auch in dem Reaktor für die Weiterbehandlung des Abwassers Verstopfungsprobleme durch suspendierte Feststoffe auftreten können, wird das Abwasser vorteilhafterweise vor einer weiteren Dechlorierungs- und/oder Dehydrochlorierungsbehandlung von suspendierten Feststoffen befreit, vorzugsweise durch chemische Reaktion und/oder mechanische Trennverfahren, die beispielsweise gemäß der bereits vorgenannten Art sein können.

Weiterhin kann es vorteilhaft sein, das Abwasser vor der Weiterbehandlung gegebenenfalls zu kühlen und/oder eine pH-Wert-Änderung vorzunehmen.

Nach einer weiteren Ausführungsform des erfindungsgemäßen Verfahrens kann die Behandlung des Abwassers gemäß dem ersten Verfahrensschritt mindestens einmal wiederholt werden.

Die Aufheizung des zu behandelnden Abwasserstroms auf die erfindungsgemäßen Behandlungstemperaturen kann in konventionellen Heizeinrichtungen erfolgen, wobei zur Aufheizung elektrische Energie oder thermische Energie, beispielsweise gespeichert in Heißdampf, verwendet werden können.

Um die Energiebilanz des erfindungsgemäßen Verfahrens weiterhin zu verbessern, ist es vorteilhaft, die in dem erhitzten, behandelten und gegebenenfalls weiterbehandelten Abwasserstrom gespeicherte Wärmeenergie zumindest teilweise auf einen noch zu behandelnden, kühleren Abwasserstrom zu übertragen, wobei gleichzeitig der heiße, behandelte Abwasserstrom gekühlt wird. Hierzu wird ein Wärmeaustauscher eingesetzt, den das behandelte und gegebenenfalls weiterbehandelte Abwasser nach dem Austragen aus dem Dechlorierungs- bzw. Dehydrochlorierungsreaktor bzw. den -reaktoren durchströmt.

Vorzugsweise erfolgt der Wärmeaustausch durch eine direkte Übertragung von Wärmeenergie über Entspannung und Kondensation, indem das heiße, unter Druck stehende, behandelte Abwasser entspannt wird, wobei insbesondere Wasserdampf entsteht, der in einen noch zu behandelnden, kühleren Abwasserstrom eingetragen wird und an diesen seine Wärmeenergie durch Kondensation abgibt.

Nach einer weiteren vorteilhaften Ausführungsform des erfindungsgemäßen Verfahrens wird die direkte Übertragung von Wärmeenergie mindestens zweistufig, vorzugsweise dreistufig, durchgeführt.

Während und/oder nach der Aufheizphase des erfindungsgemäßen Behandlungsverfahrens werden in dem zu behandelnden Abwasser Gase und/oder Dämpfe frei, insbesondere mit den leichter flüchtigen organischen Verbindungen beladener Wasserdampf. Diese Gase und/oder Dämpfe werden nach einer vorzugsweisen Ausführungsform des erfindungsgemäßen Verfahrens in den vorgeschalteten, das Abwasser produzierenden Prozeß, das heißt in den Reaktor der Epichlorhydrinsynthese zurückgeführt.

Vorzugsweise werden mit dem erfindungsgemäßen Verfahren Abwässer der Epichlorhydrinsynthese behandelt, die einen Gesamtgehalt an gelösten organischen Stoffen von mehr als 0,15 g/l aufweisen.

Weiterhin kann das in dem erfindungsgemäßen Dechlorierungs- und/oder Dehydrochlorierungsverfahren behandelte Abwasser, vorzugsweise nach einer Abkühlung, einer weiteren Reinigung, vorzugsweise einer biologischen Behandlung unter Verwendung von Mikroorganismen, unterworfen werden. Durch die gemäß der vorliegenden Erfindung vorausgegangene Reduzierung des AOX in dem Abwasser der Epichlorhydrinsynthese ist ein nachfolgender biochemischer bzw. biologischer Abbau der verbleibenden organischen Verbindungen mit Hilfe von Bakterien möglich, wobei durch den mikrobiellen Abbau gleichzeitig auch der CSB-Wert (Chemischer Sauerstoffbedarf) des Abwassers gesenkt wird, was aus einer Vernichtung der in dem Abwasser gelösten organischen Verbindungen (die als Folgeprodukte aus dem Dechlorierungs- bzw. Dehydrochlorierungsprozeß und als Nebenprodukte aus der Epichlorhydrinsynthese stammen) durch die Stoffwechseltätigkeit der Mikroorganismen resultiert.

Nach einer weiteren Ausführungsform des erfindungsgemäßen Verfahrens kann das nach der thermisch-alkalischen Behandlung aus dem Reaktor ausgetragene Abwasser auch direkt der biologischen Behandlung zugeführt werden.

Die biologische Behandlung kann im aeroben oder anaeroben Betrieb durchgeführt werden.

Vor einer biologischen Behandlung des Abwassers kann gegebenenfalls eine entsprechende Veränderung des pH-Wertes vorgenommen werden, um das Abwasser einer mikrobiologischen Behandlung zugänglich zu machen.

Eine mögliche Vorrichtung zur technischen Durchführung des erfindungsgemäßen Verfahrens und eine schematische Darstellung einer vorzugsweisen Verfahrensführung in Form eines Fließdiagramms ist in der Zeichnungsfigur 1 dargestellt.

Die Zeichnungsfigur 1 betrifft ein kontinuierliches Verfahren. Aus dem Reaktor der Epichlorhydrinsynthese (in der Zeichnungsfigur nicht dargestellt) wird über eine Pumpe **(1)** das zu behandelnde Abwasser über eine Zuleitung **A** in einen Sedimentationsabscheider **(2)** eingeführt, in dem das Abwasser von suspendierten Feststoffen zumindest teilweise befreit wird. Unter Einsätz einer weiteren Pumpe **(3)** erfolgt über eine Ableitung **C** der Sumpfabzug des Sedimentationsabscheiders. Über eine weitere Ableitung **B** wird der Klarlauf des Sedimentationsabscheiders im oberen Teil des Absetzapparates ausgetragen.

Das Abwasser passiert dann (wenn bereits ein erster Behandlungszyklus für einen Abwasserstrom durchgeführt worden ist und ein heißer, behandelter Abwasserstrom zur Verfügung steht) einen Wärmeaustauscher **(8)**, wo die Wärme eines bereits behandelten Abwasserstroms auf den unbehandelten Eingangsstrom übertragen wird, so daß eine energieeinsparende Vorheizung des Abwassers vorgenommen wird. Der heiße Abwasserstrom der als Energieträger fungiert, gelangt über Zuleitung **D** in den Wärmetauscher und wird nach der Energieübertragung, womit eine Kühlung des behandelten Abwasserstroms einhergeht, über den Ablauf **H** aus dem Wärmetauscher abgezogen und einem Ablauf **E** zur Weiterbehandlung und/oder -verwendung des Abwassers zugeführt.

Die weitere Aufheizung des unbehandelten Abwasserstroms auf die endgültige Dechlorierungs- bzw. Dehydrochlorierungstemperatur erfolgt in einer Heizeinrichtung **(9)**, die über eine Leitung **I** mit dem Wärmetauscher in Verbindung steht. Als Heizmedium kann beispielsweise Heißdampf eingesetzt werden, der über Zuleitung **J** der Heizeinrichtung zugeführt wird.

Das aufgeheizte Abwasser wird anschließend über die Zuführleitung **G** in die drei, hintereinandergeschalteten Dechlorierungs- bzw. Dehydrochlorierungs-Reaktoren **(4, 5, 6)** eingespeist, wobei das unbehandelte Abwasser jeweils am Kopf der Reaktionsgefäße eintritt und durch einen Auslaß jeweils im unteren Teil der Reaktoren ausgetragen wird. Der Betrieb des Reaktors erfolgt flutend. Das Abwasser verbleibt entsprechend der Verweilzeiteinstellung in den Reaktoren und wird nach Ende der Behandlungszeit in einen Feststoffabscheider **(10)** eingeführt, in dem das Abwasser durch Zugabe von Salzsäure auf einen solchen pH-Wert eingestellt wird, daß suspendiertes Calciumhydroxid zumindest teilweise in Lösung geht. Danach verbleibende Feststoffe werden aus dem Abwasser abgetrennt und über eine Ableitung **L** aus dem Feststoffabscheider ausgetragen. Die Zuführung der Salzsäure erfolgt über Leitung **K** mittels einer Pumpe **(12)**. Über Leitung **M** gelangt das Abwasser in einen Sprühturm **(11)** und wird dort mit gasförmigem Wasserstoff beladen, der über die Zuführung **N** in den Sprühturm gelangt. Das Abwasser-Wasserstoff-Gemisch wird über Leitung **O** in den Reaktor **(7)** eingespeist, der vorzugsweise als Wirbelschichtreaktor ausgelegt ist, und das Abwasser wird hier einer weiteren Dechlorierungs- und/oder Dehydrochlorierungsbehandlung unterworfen. Über den Ablauf **D** wird das Abwasser nach Ende der Behandlung dem Wärmetauscher **(8)** zugeführt.

Bevor oder nachdem das behandelte Abwasser den Wärmetauscher passiert bzw. passiert hat, kann eine weitere Abtrennung von suspendierten Feststoffen aus dem behandelten Abwasserstrom vorgenommen werden. Dies erfolgt vorzugsweise ebenfalls in einem Sedimentationsabscheider.

Während des Aufheizens und während der Behandlung des Abwassers bilden sich Gase bzw. Dämpfe, die vorzugsweise aus dem Abwasserstrom abgetrennt werden und über eine Ableitung **F** beispielsweise in den Reaktor der Epichlorhyhydrinsynthese zurückgeführt werden.

Des weiteren kann der Sumpfabzug **C** des Sedimentationsabscheiders **(2)** dazu benutzt werden, den pH-Wert des behandelten Abwasserstroms **E** so einzustellen, daß das Abwasser nachfolgend einer mikrobiologischen Behandlung unterworfen werden kann.

Die in den nachfolgenden Ausführungsbeispielen niedergelegten Versuche sollen die vorliegende Erfindung erläutern, ohne sie darauf zu beschränken.

### Beispiel 1

1 l eines Abwassers aus der Epichlorhydrinproduktion mit einem AOX-Gehalt von ca. 35 mg/l und einem pH-Wert von 12 (gemessen bei Raumtemperatur) wurde in einem Reaktor 3 Stunden lang bei 85°C und einem Druck von 1 bar (abs.) der erfindungsgemäßen thermisch-alkalischen Dechlorierungs- und/oder Dehydrochlorierungsbehandlung unterworfen. Der AOX-Gehalt des Abwassers konnte hierdurch um ca. 30 % vermindert werden.

Anschließend wurde durch Zugabe von Salzsäure ein pH-Wert von 10 in dem Abwasser eingestellt und verbleibende Feststoffe wurden durch Sedimentation abgetrennt. Danach wurde das Abwasser in Gegenwart von Wasserstoff und eines palladiumhaltigen Katalysators eine Stunde lang bei Raumtemperatur im Wirbelbett behandelt. Der AOX-Gehalt des Abwassers konnte hierdurch um 83 % vermindert werden.

### Beispiel 2

1 l eines Abwassers aus der Epichlorhydrinproduktion mit einem AOX-Gehalt von ca. 35 mg/l und einem pH-Wert von 12 (gemessen bei Raumtemperatur) wurde in einem Reaktor 5 Stunden lang bei 130°C und einem Druck von 2,5 bar (abs.) der erfindungsgemäßen thermisch-alkalischen Dechlorierungs- und/oder Dehydrochlorierungsbehandlung unterworfen. Der AOX-Gehalt des Abwassers konnte hierdurch um ca. 66 % vermindert werden.

Anschließend wurde durch Zugabe von Salzsäure ein pH-Wert von 10 in dem Abwasser eingestellt und verbleibende Feststoffe wurden durch Sedimentation abgetrennt. Danach wurde das Abwasser in Gegenwart von Wasserstoff und eines palladiumhaltigen Katalysators eine Stunde lang bei Raumtemperatur im Wirbelbett behandelt. Der AOX-Gehalt des Abwassers konnte hierdurch um 91 % vermindert werden.

### Beispiel 3

1 l eines Abwassers aus der Epichlorhydrinproduktion wurde analog Beispiel 2 thermisch-alkalisch behandelt und anschließend durch Zugabe von Salzsäure auf einem pH-Wert von 10,5 eingestellt, abgekühlt und einer aeroben biologischen Behandlung bei 20 °C und einer mittleren Verweilzeit von 10 Stünden unterworfen.

Durch diese Maßnahme konnte der AOX-Gehalt des Abwassers um ca. 75 % und der CSB-Wert um ca. 85 % vermindert werden.

### Beispiel 4

1 l eines Abwassers aus der Epichlorhydrinsynthese wurde analog Beispiel 2 thermisch-alkalisch und katalytisch behandelt und danach einer aeroben biologischen Behandlung bei 26 °C und einer mittleren Verweilzeit von 7 Stunden unterworfen. Der CSB-Wert konnte hierdurch um mehr als 85 % vermindert werden.

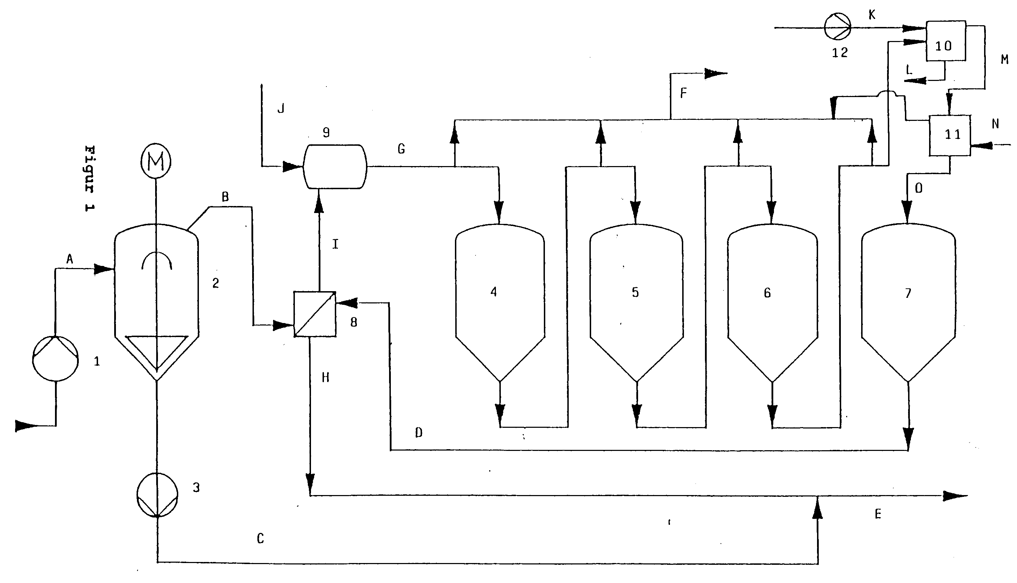

## Patentansprüche

1. Verfahren zur Behandlung von organische Stoffe, insbesondere chlororganische Verbindungen enthaltenden Abwässern aus der Epichorhydrinherstellung wobei das Abwasser, das adsorbierbare organische Halogenverbindungen AOX in einer Menge von mehr als 10 mg/l enthält und einen pH-Wert von 10 bis 14, gemessen bei Raumtemperatur, aufweist oder auf einen solchen pH-Wert eingestellt wird,
in mindestens einen Reaktor eingebracht wird und/oder diesen durchläuft, wobei eine Temperatur von mehr als 75 °C, ein Druck von mindestens 1 bar abs. und eine Verweilzeit von mindestens 0,5 Stunden in dem Reaktor eingestellt oder eingehalten werden
und danach eine weitere Dechlorierungs- und/oder Dehydrochlorierungsbehandlung in Gegenwart eines wasserstoffhaltigen Gases oder einer Wasserstoff freisetzenden Verbindung und/oder in Gegenwart einer katalytisch wirkenden Substanz durchgeführt wird, das behandelte Abwasser aus dem Reaktor oder den Reaktoren ausgetragen wird und/oder einer biologischen Behandlung unter Verwendung von Mikroorganismen unterworfen wird.

2. Verfahren zur Behandlung von organische Verbindungen enthaltenden Abwässern nach Anspruch 1, dadurch gekennzeichnet, daß das Abwasser bei einem pH-Wert von 11 bis 14 gemessen bei Raumtemperatur, einem Druck von 2 bis 10,5 bar abs., einer Verweilzeit von 1 bis 8 Stunden und einer Temperatur von 85 bis 185 °C thermisch behandelt wird und die nachfolgende Dechlorierungs- und/oder Dehydrochlorierungsbehandlung in Gegenwart von hochreinem Wasserstoff durchgeführt wird, wobei als katalytisch wirkende Substanz ein Metall, eine Metallegierung und/oder eine anorganische und/oder organische Metallverbindung oder ein Stoffgemisch, das eine oder mehrere dieser Verbindungen enthält, vorzugsweise eine palladiumhaltige Verbindung unter Zusatz eines Trägerstoffes, vorzugsweise eines aluminiumoxidhaltigen Trägerstoffes verwendet wird.

3. Verfahren zur Behandlung von organische Verbindungen enthaltenden Abwässern nach Anspruch 1 und 2, dadurch gekennzeichnet, daß das zu behandelnde Abwasser einen Gesamtgehalt von mehr als 0,15 g/l an gelösten organischen Stoffen aufweist.

4. Verfahren nach Anspruch 1 bis 3, dadurch gekennzeichnet, daß die Dechlorierungs- und/oder Dehydrochlorierungsbehandlung in einer Reaktoreinheit aus mindestens drei, vorzugsweise vier Reaktoren durchgeführt wird, wobei als Reaktor ein Strömungsrohr oder Rohrreaktor eingesetzt wird und in dem Strömungsrohr oder Rohrreaktor eine Strömungsgeschwindigkeit von mehr als 4 m/sec, vorzugsweise von 8,5 m/sec, eingestellt wird.

5. Verfahren nach einem oder mehreren der Ansprüche 1 bis 4, dadurch gekennzeichnet, daß das Abwasser vor, während und/oder nach der Dechlorierungs- und/oder Dehydrochlorierungsbehandlung von suspendierten Feststoffen, vorzugsweise durch chemische Reaktionen und/oder mechanische Trennverfahren, zumindest teilweise befreit wird.

## Claims

1. Process for treating organic substances, in particular organochlorine-compound-containing waste waters from epichlorohydrin production, characterized in that the waste water which, contains adsorbable organic halogen compounds AOX in an amount of more than 10 mg/l and has a pH of 10 to 14, measured at room temperature or is adjusted to such a pH, is introduced into at least one reactor and/or passes through this, a temperature of above 75 °C, a pressure of at least 1 bar absolute and a residence time of at least 0.5 hour being established or maintained in the reactor and then a further dechlorination treatment and/or dehydrochlorination treatment is carried out in the presence of a hydrogen-containing gas or a hydrogen-releasing compound and/or in the presence of a catalytically active substance, the treat-ed waste water is discharged from the reactor or the reactors and/or is subjected to a biological treatment using microorganisms.

2. Process for treating organic-compound-containing waste waters according to Claim 1, characterized in that the waste water is thermally treated at a pH from 11 to 14 measured at room temperature, a pressure of 2 to 10.5 bar absolute, a residence time of 1 to 8 hours and a temperature of 85 to 185 °C and the subsequent dechlorination treatment and/or dehydrochlorination treatment is carried out in the presence of highly pure hydrogen, the catalytically active substance used being a metal, a metal alloy and/or an inorganic and/or organic metal compound or a substance mixture which contains one or more of these compounds, preferably a palladium-containing compound with addition of a support material, preferably an aluminium-oxid-containing support material.

3. Process for treating organic-compound-containing waste waters according to Claim 1 and 2, characterized in that the waste water to be treated has a total content of more than 0.15 g/l of dissolved organic substances.

4. Process according to Claim 1 to 3, characterized in that the dechlorination treatment and/or dehydrochlorination treatment is carried out in a reactor unit composed of at least three, preferably four reactors, the reactor used being a flow tube or tubular reactor and in the flow tube or tubular reactor a flow velocity of more than 4m/sec, preferably 8.5 m/sec, being established.

5. Process according to one or more of Claims 1 to 4, characterized in that the waste water before, during and/or after the dechlorination treatment and/or dehydrochlorination treatment is at least partially freed from suspended solids, preferably by chemical reactions and/or mechanical separation processes.

## Revendications

1. Procédé pour le traitement d'eaux résiduaires provenant de la production d'épichlorhydrine et contenant des substances organiques, notamment des composés organiques chlorés, selon lequel l'eau résiduaire, qui contient, en une quantité supérieure à 10 mg/l, des composés halogénés organiques adsorbables (AOX) et présente une valeur de pH de 10 à 14 (mesurée à la température ambiante) ou a été ajustée à une telle valeur de pH, est introduite dans au moins un réacteur et/ou le(s) parcourt, cependant que sont ajustés et/ou maintenus dans le réacteur une température de plus de 75°C, une pression absolue d'au moins 1 bar et un temps de séjour d'au moins 0,5 heure, et qu'ensuite un traitement supplémentaire de déchloration et/ou de déshydrochloration est effectué en présence d'un gaz contenant de l'hydrogène ou d'un composé libérant de l'hydrogène et/ou en présence d'une substance à action catalytique, l'eau résiduaire traitée est retirée du réacteur ou des réacteurs et/ou l'eau résiduaire est soumise à un traitement biologique utilisant des micro-organismes.

2. Procédé pour traiter des eaux résiduaires contenant des composés organiques, selon la revendication 1, caractérisé en ce que l'eau résiduaire est soumise à un traitement thermique en étant à une valeur de pH de 11 à 14 (mesuré à la température ambiante), sous une pression absolue de 2 à 10,5 bars, pendant un temps de séjour de 1 à 8 heures et à une température de 85 à 185°C, et le traitement subséquent de déchloration et/ou de déshydrochloration est effectué en présence d'hydrogène de grande pureté, avec utilisation comme substance à action catalytique d'un métal, d'un alliage de métaux et/ou d'un composé de métal minéral et/ou organique ou d'un mélange de substances contenant un ou plusieurs de ces composés, avantageusement un composé contenant du palladium, avec addition d'une substance de support, avantageusement une substance de support contenant de l'oxyde d'aluminium.

3. Procédé selon la revendication 1 ou 2, pour traiter des eaux résiduaires contenant des composés organiques, procédé caractérisé en ce que l'eau résiduaire à traiter présente une teneur totale de plus de 0,15 g/litre en des substances organiques dissoutes

4. Procédé selon l'une des revendications 1 à 3, caractérisé en ce que le traitement de déchloration et/ou de déshydrochloration est conduit dans une unité de réacteurs consistant en au moins trois, avantageusement quatre, réacteurs, avec utilisation comme réacteur d'un tube parcouru par un écoulement ou d'un réacteur tubulaire, et l'établissement, dans le tube parcouru par un écoulement ou dans le réacteur tubulaire, d'une vitesse d'écoulement supérieure à 4 m par seconde, avantageusement de 8,5 m/s.

5. Procédé selon une ou plusieurs des revendications 1 à 4, caractérisé en ce que l'eau résiduaire est, avant, pendant et/ou après le traitement de déchloration et/ou de déshydrochloration, débarrassée au moins partiellement des solides en suspension, avantageusement par des réactions chimiques et/ou par des procédés de séparation mécanique.
